# FASCICULE DE BREVET EUROPEEN

(11) **EP 1 814 619 B1**
(45) Date de publication et mention de la délivrance du brevet: **17.08.2016**
(21) Numéro de dépôt: 05819153.7
(22) Date de dépôt: 16.11.2005
(51) Int. Cl.: A61M 15/00, B65D 75/34

(54) **DISPOSITIF DE DISTRIBUTION DE PRODUIT FLUIDE**
FLUIDPRODUKTABGABEVORRICHTUNG
FLUID PRODUCT DISPENSING DEVICE

(30) Priorité: 16.11.2004 FR 0452641
(43) Date de publication de la demande: 08.08.2007
(60) Demande divisionnaire: 15173970.3
(73) Titulaire: Aptar France SAS, 27110 Le Neubourg (FR)
(72) Inventeur: BRUNA, Pascal, F-76300 Sotteville les Rouen (FR); FOURMENT, Olivier, F-75016 Paris (FR)
(74) Mandataire: CAPRI
(86) Numéro de dépôt international: PCT/FR2005/050956
(87) Numéro de publication internationale: WO 2006/054021

(56) Documents cités:
- EP-A- 0 796 628
- EP-A- 1 106 196
- WO-A-93/15972
- WO-A-2004/011070
- WO-A-2004/041672
- WO-A2-01/00263
- DE-A1- 19 805 336
- GB-A- 2 340 758
- US-A- 5 492 112

## Description

La présente invention concerne un dispositif de distribution de produit fluide, et plus particulièrement un dispositif d'inhalation de produit pulvérulent ou poudre.

Les dispositifs d'inhalation de poudre sont bien connus dans l'état de la technique. Certain de ces dispositifs comportent un réservoir commun contenant une pluralité de doses de poudre et des moyens de dosage adaptés, à chaque actionnement, à prélever une dose de poudre destinée à être expulsée vers l'utilisateur. D'autres dispositifs prévoient des réservoirs prédosés individuels, par exemple des blisters, contenant chacun une dose unique de poudre, chaque réservoir étant ouvert individuellement lors d'un actionnement pour permettre l'expulsion du produit qu'il contient. Dans le cadre d'un dispositif avec réservoir(s) prédosé(s), différentes solutions ont été proposées pour ouvrir le réservoir et accéder au contenu afin de permettre son expulsion. Ainsi, il a été proposé de réaliser des réservoirs prédosés avec une couche pelable permettant d'exposer le réservoir et sa poudre à un écoulement de gaz, en général de l'air pour l'expulsion de la dose. D'autres dispositifs prévoient des moyens de perçage pour percer ou déchirer une partie du réservoir, permettant ainsi également d'expulser le contenu de celui-ci. Un problème qui peut se poser avec ces dispositifs concerne le risque de perte de doses, partielle ou totale, après ouverture du réservoir et avant inhalation. Ceci diminue la précision de dosage et la reproductibilité de dose (sous-dosage). Un autre problème encore plus important concerne le risque de surdosage. Ceci peut par exemple se produire si, dans un dispositif ayant des réservoirs prédosés, un réservoir est ouvert, mais l'appareil n'est pas utilisé de sorte que, le contenu du réservoir n'est pas inhalé. Dans ce cas, si du produit est susceptible de sortir du réservoir et de se déposer dans des parties du dispositif, lors du prochain actionnement, qui provoquera l'ouverture d'un autre réservoir, le risque de surdosage existe. En effet, le produit sorti du premier réservoir pourra être expulsé ensemble avec la dose complète du second réservoir. Selon la nature du produit, qui peut être un produit pharmaceutique, ce surdosage risque d'être très néfaste à l'utilisateur. Un autre problème qui se pose avec les dispositifs d'inhalation de poudre concerne la précision et la reproductibilité du dosage. En effet, il peut arriver que le vidage du réservoir ou du système de dosage ne doit pas toujours réalisé complètement à chaque actionnement, ce qui engendre des risques de différence de dosage d'une dose à l'autre, et donc une perte de précision et de reproductibilité de dosage.

Des dispositifs d'inhalation sont connus des documents WO 01/00263 et EP 1 106 196.

La présente invention a donc pour but de fournir à dispositif de distribution de produit fluide qui ne reproduit pas les inconvénients susmentionnés.

La présente invention a notamment pour but de fournir un dispositif de distribution de produit fluide qui assure une distribution précise, fiable et reproductible à chaque actionnement du dispositif.

La présente invention a encore pour but de fournir un dispositif de distribution de produit fluide qui garantit une distribution de la totalité de la dose à chaque actionnement.

La présente invention a aussi pour but de fournir un dispositif de distribution de produit fluide qui limite au maximum, voir évite, tout risque de perte de dose après ouverture du réservoir mais avant inhalation, et qui interdit tout risque de surdosage, notamment en cas d'ouverture d'un réservoir prédosé qui ne serait pas suivie d'une inhalation.

La présente invention a encore pour but de fournir un dispositif de distribution de produit fluide qui empêche tout risque de contamination du produit à distribuer.

La présente invention a également pour but de fournir un dispositif de distribution de produit fluide qui soit simple et peu coûteux à fabriquer et à assembler.

La présente invention a donc pour objet un dispositif de distribution de produit fluide tel que décrit dans la revendication 1. Des modes de réalisation avantageux sont décrits dans les revendications dépendantes. D'autres caractéristiques et avantages de la présente invention apparaîtront plus clairement au cours de la description détaillée suivante d'un mode de réalisation avantageux de celle-ci, faite en référence aux dessins joints, donnés à titre d'exemples non limitatifs, et sur lesquels :
- la figure 1 est vue schématique en section transversale d'une partie d'un dispositif de distribution de produit fluide selon un mode de réalisation avantageux de la présente invention, avant actionnement du système d'ouverture de réservoir et avant inhalation de l'utilisateur ;
- la figure 2 est une vue similaire à celle de la figure 1, après actionnement dudit système d'ouverture de réservoir et avant inhalation de l'utilisateur ;
- la figure 3 est une vue similaire à celles des figures 1 et 2, pendant l'inhalation de l'utilisateur ;
- la figure 4 est une vue similaire à celle des figures 1 à 3, après actionnement du système d'ouverture du réservoir et en l'absence d'inhalation subséquente ;
- la figure 5 est une vue schématique en section transversale d'un réservoir prédosé tel que représenté sur la figure 4 ;
- la figure 6 est une vue de dessus du réservoir de la figure 5 ; et
- la figure 7 est une vue de détail d'un élément de perçage du dispositif représenté sur les figures 1 à 4.

L'invention s'applique plus particulièrement aux dispositifs d'inhalation de produit pulvérulent ou poudre, dont un mode de réalisation avantageux sera décrit ci-après. Il est toutefois entendu que la présente invention n'est pas limitée à ce type de dispositif mais concerne de manière générale tout dispositif de distribution de produit pulvérulent.

Le dispositif d'inhalation de l'invention comporte au moins un réservoir prédosé 10 hermétiquement fermé contenant une dose unique de produit pulvérulent. Ce réservoir 10 comprend avantageusement une couche de base 11 formant une cavité recevant la poudre. Cette couche de base peut être en aluminium. Une couche de fermeture 12 est fixée à ladite couche de base 11, par exemple par collage, thermosoudure, soudure, ou tout autre moyen de fixation approprié. Cette couche de fermeture est de préférence étanche à la poudre, à l'air et à l'humidité, et peut comprendre de l'aluminium. Plusieurs réservoirs prédosés peuvent être formés sur un support commun, par exemple sous la forme d'une bande allongée, d'un disque ou d'un cylindre. Dans ce cas, le support commun peut comporter une couche de base commune 11 contenant une pluralité de cavités et une couche de fermeture commune 12, fermant hermétiquement ladite pluralité de cavités. D'autres structures sont aussi envisageables.

Le dispositif comporte en outre un canal d'expulsion 20 qui débouche dans un embout buccal (non représenté). Un système d'ouverture de réservoir 30 est prévu pour permettre d'ouvrir un réservoir prédosé 10 à chaque actionnement du dispositif.

Avantageusement, lorsque le dispositif comporte une pluralité de réservoirs prédosés, le dispositif comporte en outre des moyens de transport (non représentés) qui sont adaptés à amener un réservoir prédosé respectif 10 dans une position de distribution à chaque actionnement du dispositif pour permettre son ouverture par le système d'ouverture de réservoir 30, et l'inhalation du produit qu'il contient à travers le canal d'expulsion 20. Avantageusement, en position de distribution, le réservoir prédosé 10 coopère de manière étanche avec le canal d'expulsion 20. Ces moyens de transports pouvant être quelconque, et n'intervenant pas directement dans l'invention, ils ne seront pas décrits plus en détails ci-après.

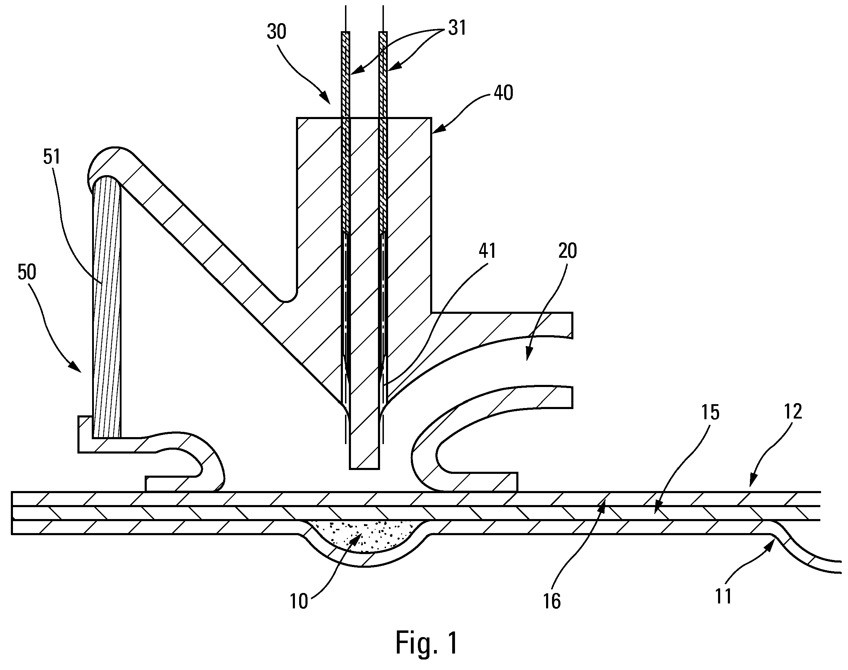

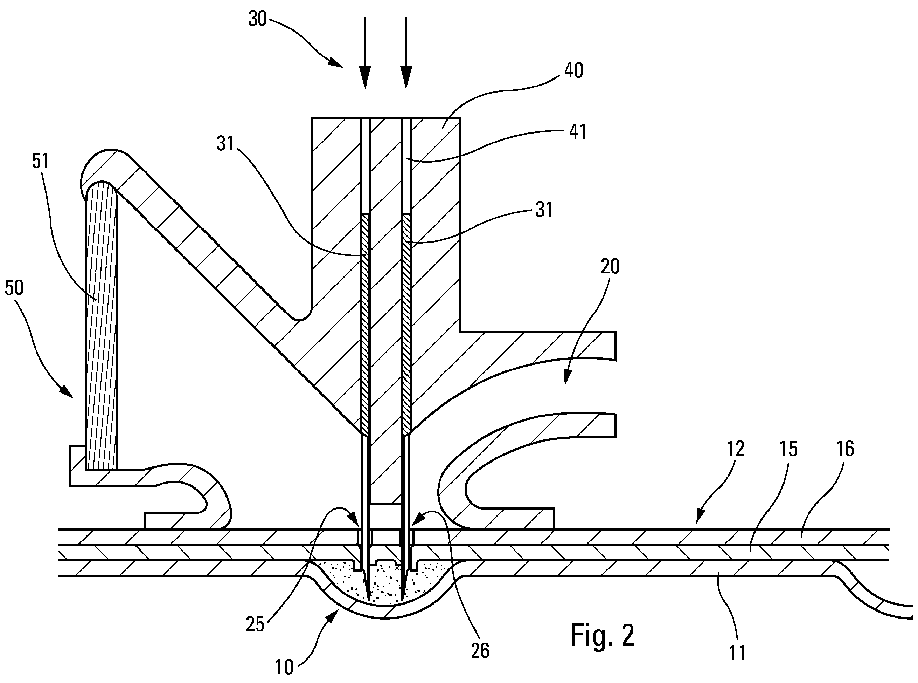

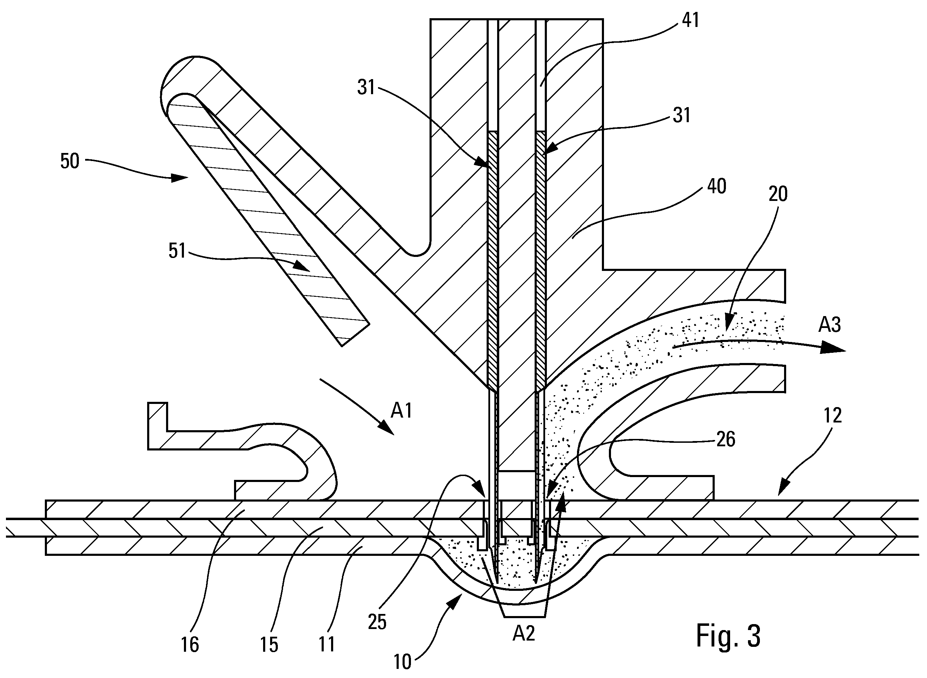

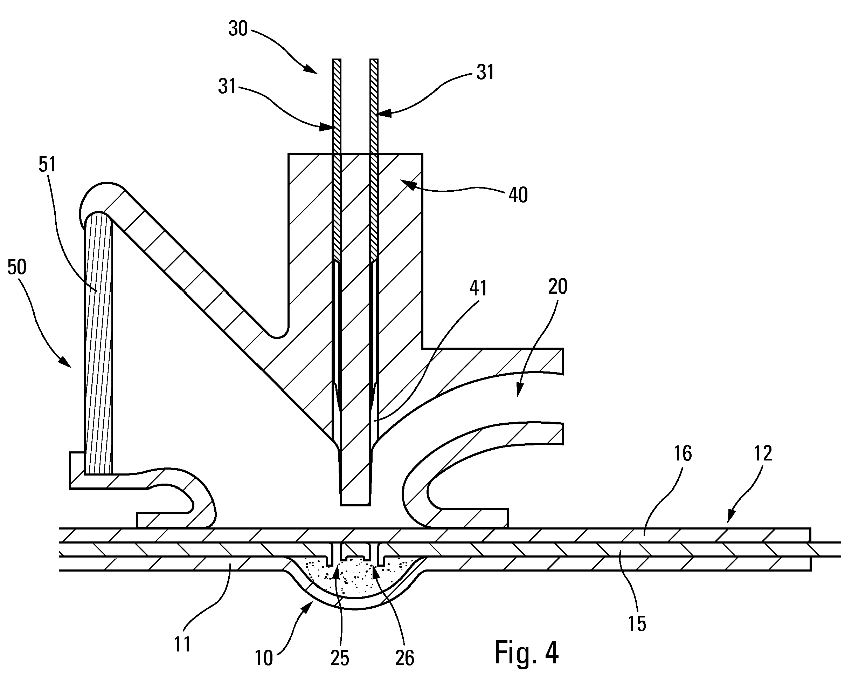

Selon l'invention, le système d'ouverture 30 comporte au moins deux éléments de perçage 31 qui sont actionnés pour créer au moins deux ouvertures distinctes dans la couche de fermeture 12 du réservoir prédosé 10. La suite de la description sera faite en relation à deux éléments de perçage 31, mais il est entendu qu'il peut y en avoir plusieurs, par exemple trois ou quatre. De préférence, ces éléments de perçage sont déplacés manuellement et simultanément, comme représenté sur les figures 1 à 4. En particulier, ces éléments de perçages 31 peuvent être déplaçables entre une position de repos, représentée sur les figures 1 et 4, et une position de perçage représentée sur les figures 2 et 3, lesdits éléments de perçage étant de préférence en position de perçage au moment de l'expulsion de la dose de produit pulvérulent hors du réservoir prédosé 10. En fait, l'extrémité de perçage de chaque élément de perçage 31 forme de préférence une partie du canal d'expulsion 20 au moment de l'expulsion. Avantageusement, le système d'ouverture 30 est actionné au moyen d'un organe de commande (non représenté) qui peut être un couvercle ou capot de protection du dispositif. Ainsi, les éléments de perçage 31 peuvent être déplacés vers leur position de perçage lors de l'ouverture du capot et ramenés vers leur position de repos lors de la fermeture dudit capot.

En se référant plus précisément aux figures 1 à 4, on constate qu'en position de distribution, le réservoir prédosé 10 coopère avec une partie amont du canal d'expulsion 20 situé en amont du réservoir 10 et une partie aval du canal d'expulsion 20 disposée en aval dudit réservoir. Au moment de l'inhalation, et dans les conditions qui sont décrites ci-après, un écoulement de gaz, notamment d'air, est créé à travers ledit canal d'expulsion 20, l'écoulement de gaz s'écoulant à partir de la partie amont du canal d'expulsion. Cet écoulement peut ensuite pénétrer à l'intérieur du réservoir prédosé 10 à travers au moins une ouverture d'entrée 25 formée par au moins un des éléments de perçage 31. L'écoulement de gaz se mélange alors avec la poudre qui est contenue de ce réservoir et l'ensemble ressort à travers au moins une seconde ouverture de sortie formée par au moins un autre élément de perçage 31, pour ensuite s'écouler dans la partie aval du canal d'expulsion 20 en direction de l'embout buccal (non représenté). Cette séquence est schématiquement représentée sur la figure 3, la flèche A1 montrant l'écoulement de gaz dans la partie amont du canal d'expulsion, la flèche A2 montrant le cheminement de l'écoulement de gaz à l'intérieur du réservoir prédosé 10, et la flèche A3 montrant l'écoulement de gaz et poudre se déplaçant en direction de l'embout buccal.

Selon l'invention, le dispositif de distribution comporte également un système de déclenchement par inhalation 50. Ce système de déclenchement par inhalation 50 comporte avantageusement un clapet 51 déplaçable entre une position de fermeture et une position d'inhalation. Dans la position de fermeture représentée sur les figures 1, 2 et 4, le clapet mobile 51 obture la partie amont du canal d'expulsion 20. De préférence, cette obturation est sensiblement étanche. De préférence, le clapet mobile 51 est sollicité vers sa position de fermeture, par tous moyens quelconque, par exemple la gravité, ou des moyens séparés, tel qu'un ressort. Le clapet mobile 51 est adapté à se déplacer vers sa position d'inhalation lorsque l'inhalation de l'utilisateur atteint un seuil prédéterminé. Tant que l'utilisateur n'inhale pas suffisamment fort pour atteindre ce seuil, le clapet mobil 51 reste fermé et aucun écoulement de gaz ne peut se propager à l'intérieur du canal d'expulsion 20, de sorte que le produit ne peut pas être expulsé hors du réservoir 10. Au moment où l'inhalation de l'utilisateur atteint le seuil prédéterminé, le clapet mobile 51 s'ouvre, par exemple pivote comme représenté sur la figure 3, ce qui provoque la création d'un écoulement de gaz, notamment d'air, qui provoquera l'expulsion du produit pulvérulent comme expliqué précédemment.

Ainsi, lors de l'expulsion du produit, les éléments de perçage 31 sont de préférence dans leur position perçage, dans laquelle ils pénètrent à l'intérieur du réservoir prédosé 10, à travers la couche de fermeture 12. Dans cette position, si l'utilisateur agite ou bouge le dispositif, les risques de perte de produit pulvérulent sont quasiment inexistants. En effet, la forme spécifique des éléments de perçage 31 rend toute sortie de poudre quasiment impossible. On pourrait aussi envisager que le canal d'expulsion comporte une forme spécifiquement adaptée pour éviter d'avantage encore les risques de perte de dose. Par exemple, des déflecteurs ou parties coudées pourraient être prévus qui retiendraient des éventuelles pertes de poudre dans le canal, de sorte que lors de l'inhalation, la totalité de la dose serait distribuée. L'invention permet donc de limiter très fortement le risque d'une perte de dose (sous-dosage) après ouverture du réservoir et avant inhalation.

Au moment de l'inhalation, l'écoulement de gaz a une puissance suffisante pour assurer un vidage total et complet du réservoir prédosé 10, en raison du seuil prédéterminé minimal que l'utilisateur doit atteindre pour provoquer l'ouverture du clapet mobile 51. L'invention permet donc de garantir un vidage complet et total et donc une précision et une reproductibilité de dosage à chaque actionnement.

Avantageusement, le dispositif comporte un corps 40 qui définit au moins partiellement le canal d'expulsion 20. Ce corps 40 peut en outre comprendre des moyens de guidage 41 pour les éléments de perçage 31 du système d'ouverture 30. Avantageusement, ce corps 40 forme également le support de l'élément de clapet mobile 51. Enfin, en position de distribution du réservoir prédosé, ce corps 40 coopère avantageusement de manière étanche avec ledit réservoir garantissant une expulsion fiable, totale, complète et reproductible à chaque actionnement. Cette mise en oeuvre permet de simplifier la fabrication et l'assemblage du dispositif, et donc d'en diminuer le coût de manière non négligeable.

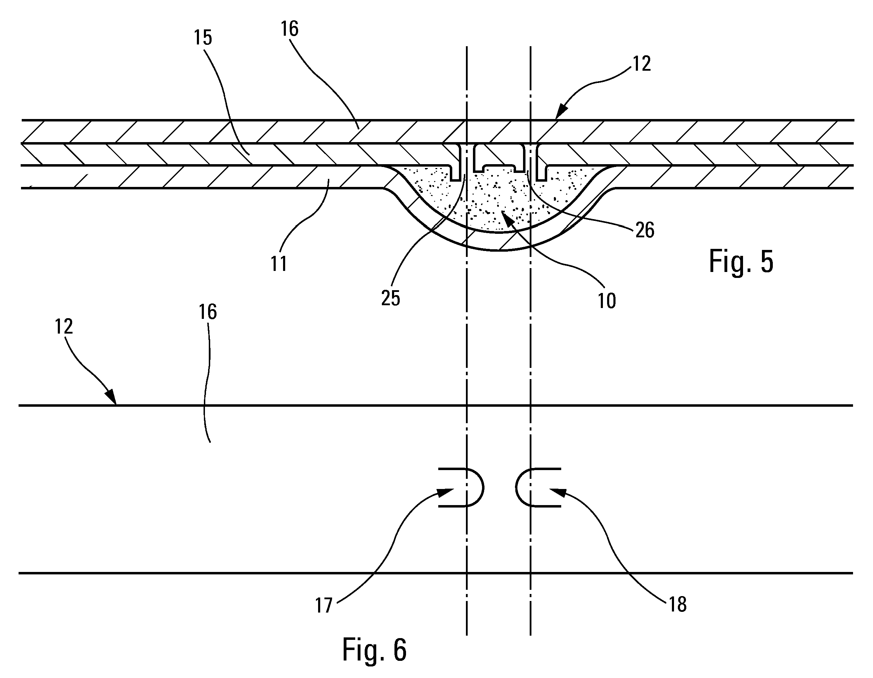

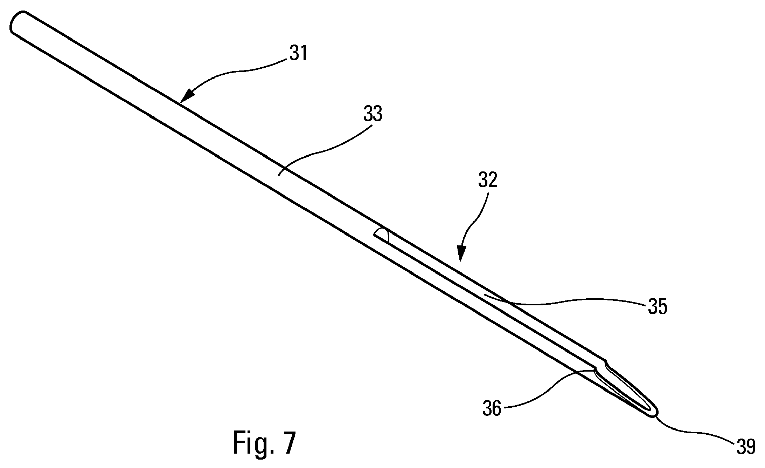

En se référant maintenant plus particulièrement à la figure 7, où il est représenté une vue de détail d'un élément de perçage 31, on constate que cet élément de perçage 31 comporte une partie de corps plein 33 et une extrémité de perçage 32 au moins partiellement creuse. C'est cette extrémité de perçage 32 qui forme une partie du canal d'expulsion 20 en position de perçage. Pour se faire, l'extrémité de perçage 32 est avantageusement réalisée sous la forme d'une pointe creuse dont une partie paroi longitudinale est découpée ou retirée pour former une ouverture longitudinale 35 sur un côté de ladite extrémité. L'exemple représenté sur la figure 7 montre une ouverture 35 formant environ un tiers de la périphérie de la pointe, mais bien entendu cette ouverture pourrait avoir des dimensions supérieures ou inférieures à celles représentées. Par exemple, l'ouverture longitudinale pourraient représentée entre environ 20% et environ 80% de la périphérie de la pointe. Par ailleurs, le bout de l'extrémité de perçage 39 est de préférence effilé, pour permettre de réaliser un perçage net et précis. Avantageusement, la dimension périphérique de l'ouverture longitudinale 35 peut également être modifiée au niveau dudit bout 39, par exemple augmentée progressivement en direction dudit bout. La partie effilée formant le bout 39 de l'extrémité de perçage 32 forme avantageusement une pente inclinée qui permet de réaliser une découpe progressive et précise au fur et à mesure que l'élément de perçage pénètre dans le réservoir 10 à travers la couche de fermeture 12. Avantageusement, un épaulement 36 peut être prévu au niveau de la jonction entre la pente inclinée du bout 39 et un bord de l'ouverture longitudinale 35, afin d'améliorer le basculement de la découpe 17, 18 formée par l'élément de perçage 31 dans la couche de fermeture 12. La figure 6 représente schématiquement une vue de dessus de la couche de fermeture 12, et montre les deux découpes 17, 18 formées par les deux éléments perçage 31. On constate que ces découpes 17, 18 restent solidaires de la couche de fermeture 12 au niveau du côté ouvert 35 de chaque élément de perçage 31. Avantageusement, les éléments de perçage 31 sont identiques, mais des différences pourraient aussi être envisagées entre le ou les élément(s) de perçage formant le ou les passage(s) d'entrée 25, et le ou les élément(s) de perçage formant le ou les passage(s) de sortie 26, par exemple au niveau de certaines dimensions.

Avantageusement, les côtés ouverts 35 des deux éléments perçage 31 sont disposés en éloignement l'un de l'autre, ce qui signifie que les côtés fermés de ces extrémités de perçage 32 se font face. Cette mise en oeuvre améliore le transport de l'écoulement de gaz vers l'intérieur du réservoir ainsi que sa sortie avec la poudre hors de celui-ci. Avantageusement, l'extrémité de perçage 32 s'étend le long de l'élément de perçage 31 sur une distance telle qu'en position de perçage (représentée sur la figure 2) le bord de l'extrémité de perçage 32 opposée au bout 39 coopère avec le corps 40, favorisant ainsi encore d'avantage l'écoulement de gaz.

Selon un aspect particulièrement avantageusement, le réservoir prédosé 10 peut comporter une couche de fermeture 12 de structure complexe. Comme décrit précédemment le réservoir 10 peut être formé par une couche de base 11, par exemple réalisé en aluminium, et qui comporte la ou les cavité(s) contenant la poudre. La couche de fermeture 12 comporte alors une couche interne 15 réalisée en un matériau étanche au produit à l'air et à l'humidité, de préférence de l'aluminium. Cette couche interne 15 est fixée sur la couche base 11 d'une manière quelconque appropriée pour fermer hermétiquement la ou les cavité(s). La couche de fermeture comporte en outre une couche externe 16 fixée à ladite couche interne 15, également d'une manière quelconque appropriée. Cette couche externe 16 est une couche de protection qui est de préférence réalisée en un matériau relativement souple et ayant une mémoire de forme, tel qu'un matériau élastomère. L'avantage de cette structure est que lorsque le système d'ouverture est actionné et que les éléments de perçage 31 viennent percer la couche de fermeture 12, des passages sont créés entre le canal d'expulsion 20 et l'intérieur du réservoir 10. Si l'utilisateur ramène le système d'ouverture 30 en position de repos, c'est-à-dire faire ressortir les éléments de perçage 31 hors du réservoir 10 pour les ramener vers leurs positions de repos respectives, la couche de protection externe 16 se referme de manière étanche pour empêcher toute perte de produit hors du réservoir. Ceci peut par exemple se produire si l'utilisateur ouvre le capot de protection de l'inhalateur et le referme sans avoir inhalé. Alors que la couche interne 15 en aluminium reste déchirée et qu'une ouverture est donc susceptible de rester à cet endroit, la couche de protection externe 16 se referme de par son élasticité en obturant ainsi le réservoir 10, de préférence de manière étanche au moins un produit pulvérulent. Ceci est particulièrement avantageusement pour éviter les risques de surdosage. En effet, en l'absence de couche de protection externe, dans l'hypothèse où l'utilisateur ouvre le réservoir puis fait ressortir le système d'ouverture sans avoir inhalé, un risque potentiel peut exister que de la poudre contenu dans ce réservoir ouvert se déverse dans le canal d'expulsion, cette poudre étant ensuite distribuée lors du prochain actionnement ensemble avec la dose complète du prochain réservoir prédosé. Ce surdosage, dans le cas de produit pharmaceutique, peut être hautement néfaste, et l'invention permet efficacement et à faible coût d'éviter ce risque. Bien entendu, la présence de la couche de protection externe 16 permet aussi d'éviter tout risque de perte de dose ou sous-dosage de manière particulièrement efficace.

Il est à noter que cette structure particulière de couche de fermeture 12, avec sa couche externe de protection, pourrait être mise en oeuvre indépendamment du dispositif d'inhalation décrit précédemment, et notamment indépendamment du système de déclenchement par l'inhalation 50. De même, une telle couche de protection externe pourrait également être efficace avec des systèmes comportant un nombre quelconque d'élément de perçage, à savoir un seul ou plusieurs.

Il est à noter que bien que l'invention ait été décrite en référence à un mode de réalisation particulier de celle-ci, elle n'est pas limitée aux exemples représentés. Ainsi, la forme du réservoir peut être quelconque. De même, les matériaux décrits ne sont donnés qu'à titre d'exemples, et d'autres matériaux sont envisageables. De plus, l'invention a été décrite en référence à l'utilisation de deux éléments de perçage 31. Il est entendu que trois ou plusieurs de tels éléments de perçage pourraient être utilisés dans le cadre de la présente invention. En particulier, on pourrait envisager un seul élément de perçage pour former l'ouverture d'entrée du réservoir et deux éléments de perçage pour former la sortie. Par ailleurs, les éléments de perçage ne sont pas nécessairement identiques et l'élément de perçage formant l'ouverture de sortie du réservoir pourrait avoir des dimensions différentes, notamment supérieures, à celle formant l'ouverture d'entrée. De plus, la structure du système de déclenchement par l'inhalation peut être quelconque, la forme du clapet 51 n'étant pas limitative.

D'autres modifications sont aussi envisageables sans sortir du cadre de la présente invention tel que définit par les revendications annexées.

## Revendications

1. Dispositif de distribution de produit fluide, comprenant au moins un réservoir prédosé (10) hermétiquement fermé et contenant une dose unique de produit pulvérulent, un canal d'expulsion (20) se terminant par un embout buccal, et un système d'ouverture de réservoir (30), chaque réservoir (10) comportant une couche de fermeture (12) apte à être percée et/ou déchirée par ledit système d'ouverture (30) pour permettre une expulsion du produit pulvérulent contenu dans ledit réservoir (10) à travers ledit canal d'expulsion (20), ledit système d'ouverture (30) de réservoir comportant au moins deux éléments de perçage (31) actionnés pour créer au moins deux ouvertures distinctes (25, 26) dans ladite couche de fermeture (12), formant au moins un passage d'entrée (25) d'écoulement de gaz, notamment d'air, et au moins un passage de sortie (26) du produit pulvérulent entraîné par ledit écoulement de gaz, et ledit dispositif comportant un système de déclenchement par l'inhalation (50) créant un écoulement de gaz dans ledit canal d'expulsion (20) seulement à partir d'un seuil d'inhalation prédéterminé lors d'une inhalation d'un utilisateur, lesdits éléments de perçage (31) étant déplaçables entre une position de repos et une position de perçage dans laquelle ils pénètrent dans un réservoir (10), lesdits éléments de perçage (31) étant en position de perçage lors de l'expulsion de la dose de produit pulvérulent hors du réservoir (10), **caractérisé en ce que** chaque élément de perçage (31) comporte une extrémité de perçage (32) au moins partiellement creuse, ladite extrémité de perçage (32) formant une partie dudit canal d'expulsion (20) à travers ladite couche de fermeture (12), en position de perçage, et à l'exception desdites extrémités de perçage (32), lesdits éléments de perçage (31) comportent un corps plein (33).

2. Dispositif selon la revendication 1, dans lequel lesdits éléments de perçage (31) sont déplacés simultanément.

3. Dispositif selon la revendication 1 ou 2, dans lequel lesdits éléments de perçage (31) sont déplacés manuellement avant l'inhalation par l'utilisateur.

4. Dispositif selon l'une quelconque des revendications précédentes, dans lequel ladite extrémité de perçage (32) est formée par une pointe creuse dont une partie de paroi longitudinale est retirée, pour former une ouverture longitudinale (35) sur un côté.

5. Dispositif selon la revendication 4, dans lequel ladite ouverture longitudinale (35) représente entre environ 20% et environ 80% de la périphérie de ladite pointe (32).

6. Dispositif selon la revendication 4 ou 5, dans lequel les ouvertures longitudinales (35) desdites extrémités de perçage (32) de deux éléments de perçage (31) sont dirigés en éloignement l'une de l'autre, les côtés fermés desdites extrémités de perçage (32) étant disposés l'un face à l'autre.

7. Dispositif selon l'une quelconque des revendications 4 à 6, dans lequel le bout de perçage (39) de chaque extrémité de perçage (32) est effilé.

8. Dispositif selon l'une quelconque des revendications 4 à 7, dans lequel la dimension périphérique de l'ouverture longitudinale (35) augmente progressivement en direction dudit bout (39).

9. Dispositif selon l'une quelconque des revendications 4 à 8, dans lequel, lorsque les éléments de perçage (31) sont actionnés, chaque élément de perçage (31) réalise une découpe respective (17, 18) dans ladite couche de fermeture (12), ladite découpe (17, 18) restant solidaire de ladite couche de fermeture (12) au niveau du côté ouvert (35) de chaque extrémité de perçage (32).

10. Dispositif selon l'une quelconque des revendications précédentes, dans lequel ledit système de déclenchement par l'inhalation (50) comporte un clapet mobile (51) dans le canal d'expulsion (20), en amont dudit réservoir (10), ledit clapet mobile (51) étant sollicité vers une position de fermeture obturant ledit canal d'expulsion (20) et se déplaçant vers une position d'inhalation, dans laquelle il ouvre ledit canal d'expulsion (20), permettant à un écoulement de gaz de s'écouler en direction dudit réservoir (10) puis dudit embout buccal du dispositif, lorsque l'inhalation de l'utilisateur atteint ou dépasse ledit seuil prédéterminé.

11. Dispositif selon la revendication 10, dans lequel ledit clapet mobile (51) obture le canal d'expulsion (20) de manière sensiblement étanche en position de fermeture.

12. Dispositif selon l'une quelconque des revendications précédentes, dans lequel ledit dispositif comporte un corps (40) définissant au moins partiellement ledit canal d'expulsion (20), ledit corps (40) comportant des moyens de guidage (41) desdits éléments de perçage (31).

13. Dispositif selon les revendications 10 et 12, dans lequel ledit corps (40) supporte ledit clapet mobile (51).

14. Dispositif selon l'une quelconque des revendications précédentes, comportant une pluralité de réservoirs prédosés (10), ledit dispositif comportant des moyens de transport pour, à chaque actionnement du dispositif, transporter un réservoir prédosé (10) respectif vers une position de distribution, dans laquelle il est disposé à proximité dudit canal d'expulsion (20) et dudit système d'ouverture (30), permettant un actionnement dudit système d'ouverture (30) et une inhalation de l'utilisateur pour réaliser l'expulsion d'une dose de produit pulvérulent.

15. Dispositif selon la revendication 14, dans lequel ladite pluralité de réservoirs prédosés (10) est formée sur un support commun, telle qu'une bande allongée, un disque ou cylindre.

16. Dispositif selon la revendication 14 ou 15, dans lequel, en position de distribution, chaque réservoir prédosé (10) coopère avec ledit canal d'expulsion (20) de manière sensiblement étanche.

17. Dispositif selon l'une quelconque des revendications 14 à 16, comprenant un organe de commande, tel qu'un couvercle du dispositif, adapté à actionner d'une part le système d'ouverture (30) de réservoir et d'autre part lesdits moyens de transport.

18. Dispositif selon l'une quelconque des revendications précédentes, dans lequel ledit canal d'expulsion (20), en aval dudit réservoir prédosé (10) dans le sens de l'écoulement de gaz, a une forme adaptée à au moins limiter le risque de perte de produit fluide en l'absence d'inhalation après actionnement du système d'ouverture (30).

19. Dispositif selon l'une quelconque des revendications précédentes, dans lequel chaque réservoir prédosé (10) comporte une couche de base (11) comportant une cavité et une couche de fermeture (12) fixée à la couche de base (11) pour fermer hermétiquement ladite cavité.

20. Dispositif selon la revendication 19, dans lequel ladite couche de fermeture (12) comporte une couche interne (15) en matériau étanche au produit, à l'air et à l'humidité, telle qu'une feuille d'aluminium, et une couche externe de protection (16) fixée sur ladite couche interne (15).

21. Dispositif selon la revendication 20, dans lequel ladite couche externe (16) est réalisée en un matériau ayant une mémoire de forme et sensiblement élastique, tel qu'un matériau élastomère, de sorte que si les éléments de perçage (31) du système d'ouverture (30) sont actionnés, puis ramenés dans leur position de repos sans que le produit pulvérulent contenu dans le réservoir (10) ait été expulsé, la couche externe (16) se referme de manière étanche au niveau des découpes (17, 18) formées par lesdits éléments de perçage (31) empêchant toute sortie de produit pulvérulent dudit réservoir (10) vers ledit canal d'expulsion (20).

22. Dispositif selon l'une quelconque des revendications 19 à 21, dans lequel plusieurs réservoirs prédosés sont formés sur un support commun, comportant une couche de base commune (11) pourvue d'une pluralité de cavités et une couche de fermeture commune (12) fermant hermétiquement ladite pluralité de cavités.

## Patentansprüche

1. Ausgabevorrichtung für ein fluides Produkt, aufweisend mindestens einen vordosierten Behälter (10), der hermetisch verschlossen ist und eine einzelne Dosis eines pulverförmigen Produktes enthält, einen Austreibungskanal (20), der in einem Mundansatz mündet, und ein Öffnungssystem (30) für den Behälter, wobei jeder Behälter (10) eine Verschlussschicht (12) aufweist, die durch das Öffnungssystem (30) durchstoßen und/oder zerrissen werden kann, um ein Austreiben des in dem Behälter (10) enthaltenen pulverförmigen Produktes durch den Austreibungskanal (20) zu ermöglichen, wobei das Öffnungssystem (30) für den Behälter (30) mindestens zwei Durchstoßelemente (31) aufweist, die betätigt werden, um mindestens zwei getrennte Öffnungen (25, 26) in der Verschlussschicht (12) zu schaffen, die mindestens einen Einlassdurchgang (25) für einen Gasstrom, insbesondere einen Luftstrom, und mindestens einen Auslassdurchgang (26) für das pulverförmige Produkt, das durch den Gasstrom mitgerissen wird, bilden, und wobei die Vorrichtung ein durch Inhalation ausgelöstes System (50) aufweist, welches bei einer Inhalation durch einen Benutzer nur über einer vorbestimmten Inhalationsschwelle einen Gasstrom in dem Austreibungskanal (20) schafft, wobei die Durchstoßelemente (31) zwischen einer Ruheposition und einer Durchstoßposition, in der sie in einen Behälter (10) eindringen, verschiebbar sind, wobei sich die Durchstoßmittel (31) während des Austreibens der Dosis von pulverförmigem Produkt aus dem Behälter (10) in der Durchstoßposition befinden, **dadurch gekennzeichnet dass** jedes Durchstoßelement (31) ein Durchstoßende (32) aufweist, welches zumindest teilweise hohl ist, wobei das Durchstoßende (32) in der Durchstoßposition einen Teil des Austreibungskanals (20) durch die Verschlussschicht (12) bildet und die Durchstoßelemente (31) mit Ausnahme der Durchstoßenden (32) einen massiven Körper (33) aufweisen.

2. Vorrichtung nach Anspruch 1, wobei die Durchstoßelemente (31) gleichzeitig verschoben werden.

3. Vorrichtung nach Anspruch 1 oder 2, wobei die Durchstoßelemente (31) vor der Inhalation durch den Benutzer manuell verschoben werden.

4. Vorrichtung nach einem der vorhergehenden Ansprüche, wobei das Durchstoßende (32) durch eine hohle Spitze gebildet ist, von welcher ein Wandteil in Längsrichtung zurückgezogen ist, um eine Längsöffnung (35) auf einer Seite zu bilden.

5. Vorrichtung nach Anspruch 4, wobei die Längsöffnung (35) zwischen ungefähr 20 % und ungefähr 80 % des Umfangs der Spitze (32) darstellt.

6. Vorrichtung nach Anspruch 4 oder 5, wobei die Längsöffnungen (35) der Durchstoßenden (32) von zwei Durchstoßelementen (31) voneinander weg ausgerichtet sind, wobei die geschlossenen Seiten der Durchstoßenden (32) einander gegenüberliegend angeordnet sind.

7. Vorrichtung nach einem der Ansprüche 4 bis 6, wobei das äußerste Durchstoßende (39) eines jeden Durchstoßendes (32) konisch zuläuft.

8. Vorrichtung nach einem der Ansprüche 4 bis 7, wobei die Umfangsabmessung der Längsöffnung (35) in Richtung zum äußersten Ende (39) allmählich zunimmt.

9. Vorrichtung nach einem der Ansprüche 4 bis 8, wobei, wenn die Durchstoßelemente (31) betätigt werden, jedes Durchstoßelement (31) ein jeweiliges abgeschnittenes Teil (17, 18) in der Verschlussschicht (12) erzeugt, wobei das abgeschnittene Teil (17, 18) mit der Verschlussschicht (12) an der offenen Seite (35) eines jeden Durchstoßendes (32) verbunden bleibt.

10. Vorrichtung nach einem der vorhergehenden Ansprüche, wobei das durch Inhalation ausgelöste System (50) eine bewegliche Klappe (51) in dem Austreibungskanal (20) stromaufwärts des Behälters (10) aufweist, wobei die bewegliche Klappe (51) in eine Verschlussposition vorgespannt ist, die den Austreibungskanal (20) absperrt, und sich in eine Inhalationsposition verschiebt, in der sie den Austreibungskanal (20) öffnet und dadurch ermöglicht, dass ein Gasstrom in Richtung des Behälters (10) und dann aus dem Mundansatz der Vorrichtung strömen kann, wenn die Inhalation durch den Benutzer die vorbestimmte Schwelle erreicht oder überschreitet.

11. Vorrichtung nach Anspruch 10, wobei die bewegliche Klappe (51) den Austreibungskanal (20) in der Verschlussposition im Wesentlichen dichtend absperrt.

12. Vorrichtung nach einem der vorhergehenden Ansprüche, wobei die Vorrichtung einen Körper (40) aufweist, der den Austreibungskanal (20) zumindest teilweise definiert, wobei der Körper (40) Führungsmittel (41) der Durchstoßelemente (31) aufweist.

13. Vorrichtung nach den Ansprüchen 10 und 12, wobei der Körper (40) die bewegliche Klappe (51) trägt.

14. Vorrichtung nach einem der vorhergehenden Ansprüche, aufweisend mehrere vordosierte Behälter (10), wobei die Vorrichtung Transportmittel aufweist, um bei jeder Betätigung der Vorrichtung einen vordosierten Behälter (10) jeweils in eine Ausgabeposition zu transportieren, in der er in der Nähe des Austreibungskanals (20) und des Öffnungssystems (30) angeordnet ist, wodurch eine Betätigung des Öffnungssystems (30) und eine Inhalation durch den Benutzer, um die Austreibung einer pulverförmigen Produktdosis zu bewirken, ermöglicht wird.

15. Vorrichtung nach Anspruch 14, wobei die mehreren vordosierten Behälter (10) durch einen gemeinsamen Träger gebildet sind, wie durch einen länglichen Streifen, eine Scheibe oder einen Zylinder.

16. Vorrichtung nach Anspruch 14 oder 15, wobei in der Ausgabeposition jeder vordosierte Behälter (10) im Wesentlichen dichtend mit dem Austreibungskanal (20) zusammenwirkt.

17. Vorrichtung nach einem der Ansprüche 14 bis 16, aufweisend ein Steuerelement, wie einen Deckel der Vorrichtung, das in der Lage ist, einerseits das Öffnungssystem (30) des Behälters und andererseits die Transportmittel zu betätigen.

18. Vorrichtung nach einem der vorhergehenden Ansprüche, wobei der Austreibungskanal (20) stromabwärts des vordosierten Behälters (10) in Richtung des Gasstroms eine Form aufweist, die derart ist, dass sie das Risiko des Verlustes von fluidem Produkt bei fehlender Inhalation nach der Betätigung des Öffnungssystems (30) zumindest begrenzt.

19. Vorrichtung nach einem der vorhergehenden Ansprüche, wobei jeder vordosierte Behälter (10) eine Grundschicht (11), die einen Hohlraum aufweist, und eine Verschlussschicht (12) aufweist, die an der Grundschicht (11) befestigt ist, um den Hohlraum hermetisch abzuschließen.

20. Vorrichtung nach Anspruch 19, wobei die Verschlussschicht (12) eine innere Schicht (15) aus einem gegenüber dem Produkt, gegenüber Luft und Feuchtigkeit dichtem Material, wie einer Aluminiumfolie, und eine äußere Schutzschicht (16), die an der inneren Schicht (15) befestigt ist, aufweist.

21. Vorrichtung nach Anspruch 20, wobei die äußere Schicht (16) aus einem Material gefertigt ist, welches ein Formgedächtnis hat und im Wesentlichen elastisch ist, wie ein elastomeres Material, so dass, wenn die Durchstoßelemente (31) des Öffnungssystems (30) betätigt und dann in ihre Ruheposition zurückgeführt werden, ohne dass das in dem Behälter (10) enthaltene pulverförmige Produkt ausgetrieben wurde, sich die äußere Schicht (16) an den abgeschnittenen Teilen (17, 18), die durch die Durchstoßelemente (31) gebildet wurden, wieder dichtend verschließt und dadurch jeglichen Austritt von pulverförmigem Produkt aus dem Behälter (10) in Richtung zum Austreibungskanal (20) verhindert.

22. Vorrichtung nach einem der Ansprüche 19 bis 21, wobei mehrere vordosierte Behälter auf einem gemeinsamen Träger gebildet sind, aufweisend eine gemeinsame Grundschicht (11), die mit mehreren Hohlräumen versehen ist, und eine gemeinsame Verschlussschicht (12), die die mehreren Hohlräume hermetisch verschließt.

## Claims

1. A fluid dispenser device comprising: at least one predosed reservoir (10) that is hermetically sealed and that contains a single dose of powder; an expulsion channel (20) that is terminated by a mouthpiece; and a reservoir opening system (30), each reservoir (10) including a closure layer (12) that is suitable for being pierced and/or torn by said opening system (30) so as to enable the powder that is contained in said reservoir (10) to be expelled through said expulsion channel (20), said reservoir opening system (30) comprising at least two piercer elements (31) that are actuated so as to create at least two distinct openings (25, 26) in said closure layer (12), forming at least one inlet passage (25) for the flow of gas, in particular air, and at least one outlet passage (26) for the powder entrained by said flow of gas, said device including an inhalation trigger system (50) that creates a flow of gas in said expulsion channel (20) only when a user inhales at or above a predetermined inhalation threshold, said piercer elements (31) being displaceable between a rest position and a piercing position in which they penetrate into a reservoir (10), said piercer elements (31) being in the piercing position while the dose of powder is being expelled from the reservoir (10), **characterized in that** each piercer element (31) includes a piercing end (32) that is hollow at least in part, said piercing end (32) forming, in the piercing position, a portion of said expulsion channel (20) through said closure layer (12), and **in that** with the exception of said piercing ends (32), said piercer elements (31) comprise a solid body (33).

2. A device according to claim 1, in which said piercer elements (31) are displaced simultaneously.

3. A device according to claim 1 or claim 2, in which said piercer elements (31) are displaced manually before the user inhales.

4. A device according to any preceding claim, in which said piercing end (32) is formed by a hollow point having a longitudinal wall portion that is removed so as to form a longitudinal opening (35) on one side.

5. A device according to claim 4, in which said longitudinal opening (35) represents about 20% to about 80% of the periphery of said point (32).

6. A device according to claim 4 or claim 5, in which the longitudinal openings (35) of said piercing ends (32) of two piercer elements (31) are directed away from each other, the closed sides of said piercing ends (32) being disposed facing each other.

7. A device according to any one of claims 4 to 6, in which the piercing tip (39) of each piercing end (32) is tapering.

8. A device according to any one of claims 4 to 7, in which the peripheral dimension of the longitudinal opening (35) progressively increases towards said tip (39).

9. A device according to any one of claims 4 to 8, in which, when the piercer elements (31) are actuated, each piercer element (31) cuts out a respective flap (17, 18) in said closure layer (12), said flap (17, 18) remaining secured to said closure layer (12) at the open side (35) of each piercing end (32).

10. A device according to any preceding claim, in which said inhalation trigger system (50) comprises a movable valve member (51) in the expulsion channel (20), upstream from said reservoir (10), said movable valve member (51) being urged towards a closed position that closes said expulsion channel (20), and being displaced towards an inhaling position, in which it opens said expulsion channel (20), enabling a flow of gas to flow towards said reservoir (10) and then towards said mouthpiece of the device, when the user inhales at or beyond said predetermined threshold.

11. A device according to claim 10, in which said movable valve member (51) closes the expulsion channel (20) in substantially sealed manner in the closed position.

12. A device according to any preceding claim, in which said device includes a body (40) that defines said expulsion channel (20) at least in part, said body (40) including guide means (41) for guiding said piercer elements (31).

13. A device according to claims 10 and 12, in which said body (40) supports said movable valve member (51).

14. A device according to any preceding claim, including a plurality of predosed reservoirs (10), said device including conveyer means for acting, each time the device is actuated, to convey a respective predosed reservoir (10) to a dispensing position in which it is disposed in the proximity of said expulsion channel (20) and of said opening system (30), enabling said opening system (30) to be actuated and the user to inhale so as to expel a dose of powder.

15. A device according to claim 14, in which said plurality of predosed reservoirs (10) is formed on a single support, such as an elongate strip, a disk, or a cylinder.

16. A device according to claim 14 or claim 15, in which, in the dispensing position, each predosed reservoir (10) co-operates with said expulsion channel (20) in substantially sealed manner.

17. A device according to any one of claims 14 to 16, including a control member, such as a lid of the device, that is adapted to actuate firstly the reservoir opening system (30), and secondly said conveyor means.

18. A device according to any preceding claim, in which said expulsion channel (20), downstream from said predosed reservoir (10) in the gas-flow direction, has a shape that is adapted at least to limit the risk of loss of fluid in the absence of inhalation after the opening system (30) has been actuated.

19. A device according to any preceding claim, in which each predosed reservoir (10) comprises a base layer (11) including a cavity, and a closure layer (12) that is bonded to the base layer (11) so as to seal said cavity hermetically.

20. A device according to claim 19, in which said closure layer (12) comprises an inner layer (15) that is made of a material that is powder-tight, air-tight, and moisture-tight, such as a sheet of aluminum, and a protective outer layer (16) that is bonded on said inner layer (15).

21. A device according to claim 20, in which said outer layer (16) is made of a material that has shape memory and that is substantially elastic, such as an elastomer material, so that if the piercer elements (31) of the opening system (30) are actuated, and then returned to their rest position without the powder that is contained in the reservoir (10) having been expelled, the outer layer (16) closes in sealed manner at the flaps (17, 18) that are cut out by said piercer elements (31), preventing any powder from escaping from said reservoir (10) towards said expulsion channel (20).

22. A device according to any one of claims 19 to 21, in which a plurality of predosed reservoirs are formed on a single support that comprises a single base layer (11) provided with a plurality of cavities, and a single closure layer (12) that hermetically seals said plurality of cavities.
